# EUROPEAN PATENT SPECIFICATION

(11) **EP 4 066 873 B1**
(45) Date of publication and mention of the grant of the patent: **18.03.2026**
(21) Application number: 21166669.8
(22) Date of filing: 01.04.2021
(51) Int. Cl.: A61M 5/32

(54) **A SAFETY NEEDLE DEVICE**
SICHERHEITSNADELVORRICHTUNG
DISPOSITIF D'AIGUILLE DE SÉCURITÉ

(43) Date of publication of application: 05.10.2022
(73) Proprietor: HTL-Strefa S.A., 95-035 Ozorków (PL)
(72) Inventor: OSAK, Robert, 95-035 Ozorków (PL); KOMUDA, Marcin, 95-035 Ozorków (PL); NIEMIEC, Marcin, 95-035 Ozorków (PL); GRZELAK, Robert, 95-035 Ozorków (PL); PERLAK, Rafal, 95-035 Ozorków (PL); BIALAS, Bartlomiej, 95-035 Ozorków (PL)
(74) Representative: Perani & Partners S.p.A.

(56) References cited:
- EP-A1- 3 626 290
- EP-A1- 3 815 727
- EP-B1- 3 626 290
- WO-A1-2009/114762
- WO-A1-2020/001655

## Description

### Field of Invention

The invention relates to the technical field of a medical device, in particular to a protection device able to prevent the user from accidental or intentional injuries with a needle before and after the use of the medical device.

### Background of the Invention

Typically, a safety needle device comprises an elongated needle having a first distal end insertable into the patient's skin and a second proximal end insertable into a pen injector for supplying a substance. The needle is embedded in a hub and partially surrounded by a housing fixed to the hub. A known device comprises also a front-end shield and a back-end shield surrounding the needle and moving along the needle to protect the needle end before and after use. The shields are activated by means of spring elements interposed between the hub and the relative shield. The activation of the front-end shield occurs with the injection, while the activation of the back end shield occurs when coupling of the safety device with a pen injector. Upon activation, the shields pass into a protect configuration that prevents further uses or injuries. In detail, the device comprises locking means able to lock the sliding of the front and back-end shields after use.

For example, in the art elastic clamps engage the back-end shield when it reaches a specific position sliding towards the proximal end of the needle. Other known locking means are flexible tabs formed on the hub that engage the back-end shield to prevent further movements of it after use.

Pertinent safety needle device are known from WO 2009/114762, EP 3 626 290 and WO 2020/001655.

However, the known safety needle devices show some drawbacks. In fact, such devices do not ensure the correct protection to the user from further uses and from accidental injuries. Furthermore, known devices are complex since they require a lot of elements in order to achieve the correct protection of the user, thereby increasing the overall costs of production.

Furthermore, the structural complexity of known safety needle devices increases the production costs and the assemblage time.

### Summary of the invention

In this context, the technical task underlying the present invention is to propose a safety needle device which overcomes the drawbacks of the above prior art.

Specifically, it is an object of the present invention to provide a safety needle device able to ensure the protection to the user and at the same time to improve the availability of the device itself and to reduce the complexity of the back-end mechanism.

The technical task set out and the specified objects are substantially achieved by a cooperation of protrusions formed on the back-end shield and passing through guides formed on the hub to guide and lock the back-end shield.

The invention is defined in claim 1. Advantageous embodiments are subject of the dependent claims.

### Brief description of the drawings

The present invention will now be described in more detail hereinafter with reference to the accompanying drawings, in which some embodiments of the invention are shown.
FIG. 1 is a section view of the safety needle is a lateral view of the safety needle device according to a first embodiment of the present invention, in pre-use configuration;
FIG. 2 is a different section view of the safety needle of FIG. 1;
FIG. 3 is a section view of the safety needle device according to the embodiment of FIG. 1, in a use configuration;
FIG. 4 is a different section view of the safety needle device of FIG. 3;
FIG. 5 is a section view of the safety needle device according to the embodiment of FIG. 1, in a locking configuration;
FIG. 6 is a section view of the safety needle device according to the embodiment of FIG. 1, in an after-use configuration;
FIG. 7 is a different section view of the safety needle device of FIG. 6;
FIG. 8 is a lateral view of the safety needle device according to the embodiment of FIG. 1 where some elements are hided to better show other aspects;
FIG. 9 is a perspective view of an element of safety needle device of FIG. 1;
FIG. 10 is a section view of the safety needle device according to a second embodiment of the present invention, in pre-use configuration;
FIG. 11 is a different section view of the safety needle of FIG. 10;
FIG. 12 is a section view of the safety needle device according to the embodiment of FIG. 10, in a use configuration;
FIG. 13 is a different section view of the safety needle device of FIG. 10;
FIG. 14 is a section view of the safety needle device according to the embodiment of FIG. 10, in a locking configuration;
FIG. 15 is a section view of the safety needle device according to the embodiment of FIG. 10, in an after-use configuration;
FIG. 16 is a different section view of the safety needle device of FIG. 15;
FIG. 17 is a lateral view of the safety needle device according to the embodiment of FIG. 10 where some elements are hided to better show other aspects;
FIG. 18 is a perspective view of an element of safety needle device of FIG. 10;
FIG. 19 is a perspective view of an element of safety needle device of FIG. 1 and FIG. 10.

### Detailed description

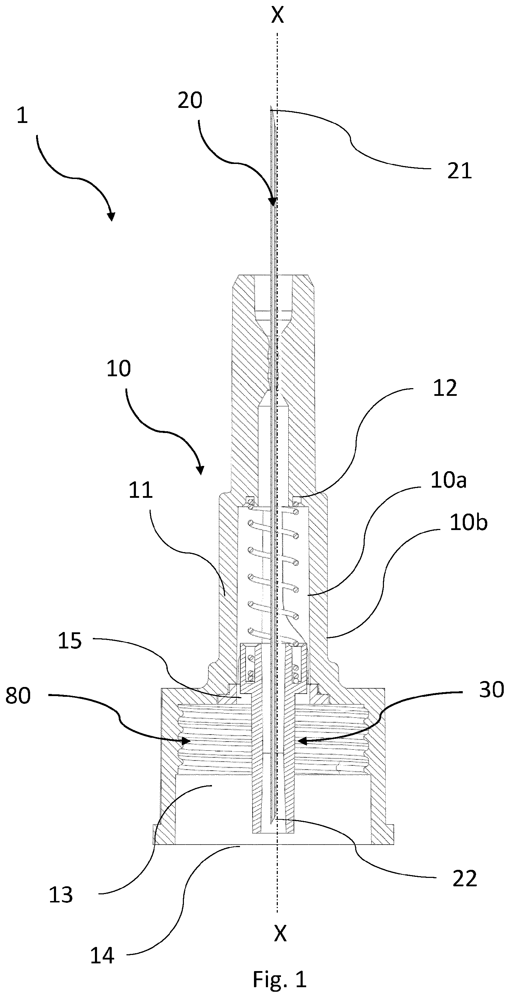

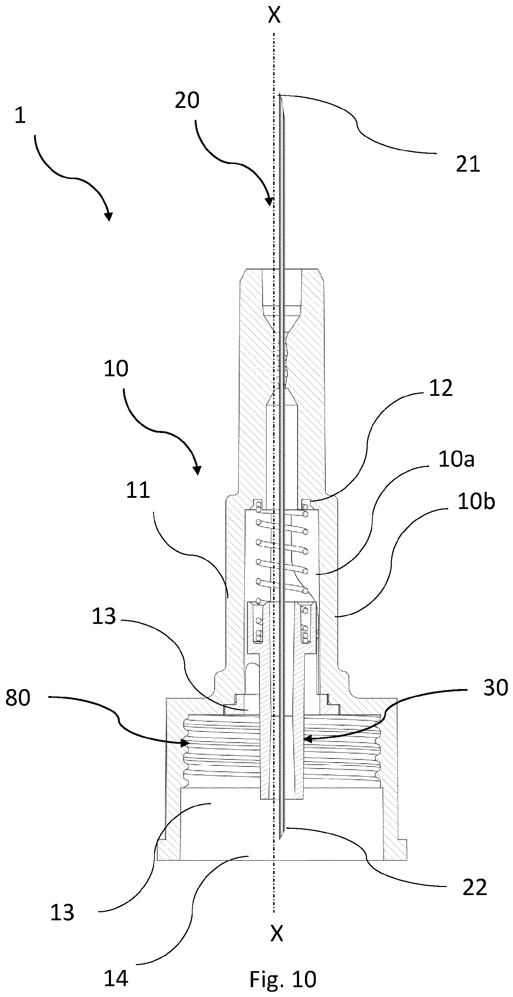

With reference to figures 1-8 and 10-17, a safety needle device is indicated as a whole by numeral 1. Specifically, the figures 1-8 and 10-17 do not show a front-end mechanism but they are focused on a back-end mechanism. Safety needle device 1 can be associated to a pen injector, not shown, containing a substance to inject into a patient. It is to be noted, as clarify in the following, that figures 1-9 show a first embodiment of the safety needle device 1 and figure 10-18 show a second embodiment of the safety needle device 1.

The safety needle device 1 comprises a hub 10 configured to be connected to the pen injector. The hub 10 comprises a lateral wall 11 and an upper wall 12 which define therebetween a cavity 13. The latest extends between the upper wall 12 and a lower aperture 14 configured to receive the pen injector.

The hub 10 has an intermediate aperture 15 defined between the upper wall 12 and the lower aperture 14.

Preferably the hub 10 has an inner surface 10a facing to the cavity 13 and opposed outer surface 10b.

More preferably, the hub 10 comprises retaining means 80 configured to retain the pen injector. The retaining means 80 are formed on the inner surface 10a of the lateral wall 11 near the aperture 14.

According to a preferred embodiment, the hub 10 has a tubular shape with a circular cross section. Preferably, the intermediate aperture 15 has a radius shorter than the radius of the lower aperture 14.

The safety needle device 1 comprises a needle 20 attached to the hub 10. The needle 20 extends along a longitudinal direction X-X between a distal end 21, formed to be inserted into a patient, and opposed proximal end 22 formed to be connected to the pen injector.

According to one embodiment, at least a portion of the needle 20 is arranged inside the cavity 13. Preferably, the proximal end 22 of the needle 20 is arranged inside the cavity 13.

The safety needle device 1 comprises a back-end shield 30 slidable along the longitudinal direction X-X within the cavity 30 of the hub 10 and rotatable about the longitudinal direction X-X. Specifically, the back-end shield 30 is slidable and rotatable with respect to the hub 10. Preferably, the back-end shield 30 is movable irreversibly from an initial position, before connection of the pen injector to the hub 10, to a locking position, after removal of the pen injector to the hub 10 passing through an intermediate position. More preferably, during the connection of the hub 10 to the pen injector, the back-end shield 30 passes from the initial position to the intermediate position sliding and rotating towards the distal end 22 of the needle 20 along and about the longitudinal direction X-X. Moreover, during the removal of the pen injector the back-end shield 30 passes from the intermediate position to the locking position sliding and rotating towards the proximal end 21 of the needle 10 along the longitudinal axis X-X.

According to a preferred embodiment, the back-end shield 30 is configured to cover and protect the proximal end 22 of the needle 30 in the locking position. Specifically, the back-end shield 30 is configured to be locked in the locking position avoiding further sliding that would lead to uncovering the proximal end 22 of the needle 20 after use.

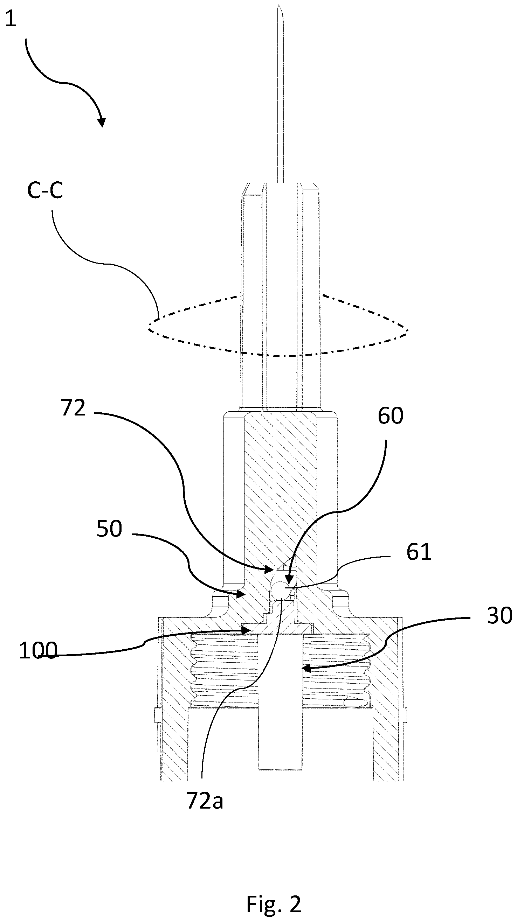

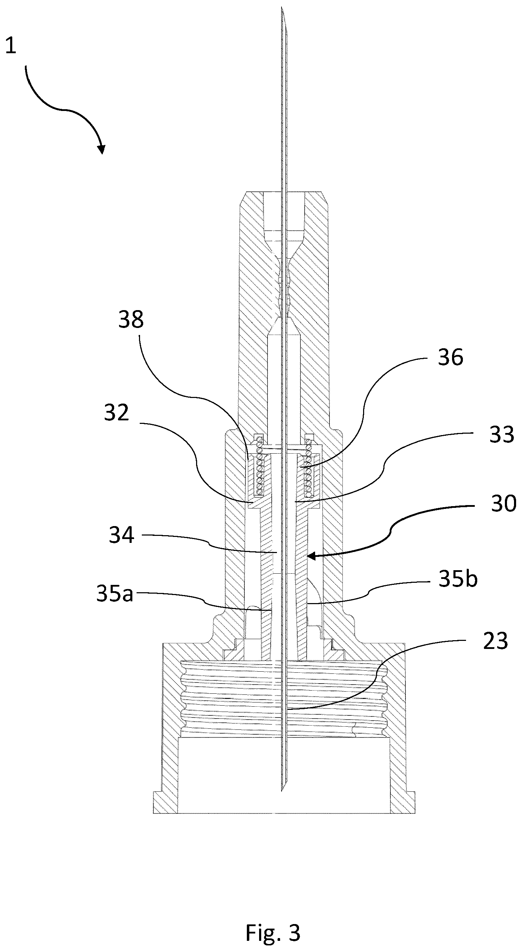

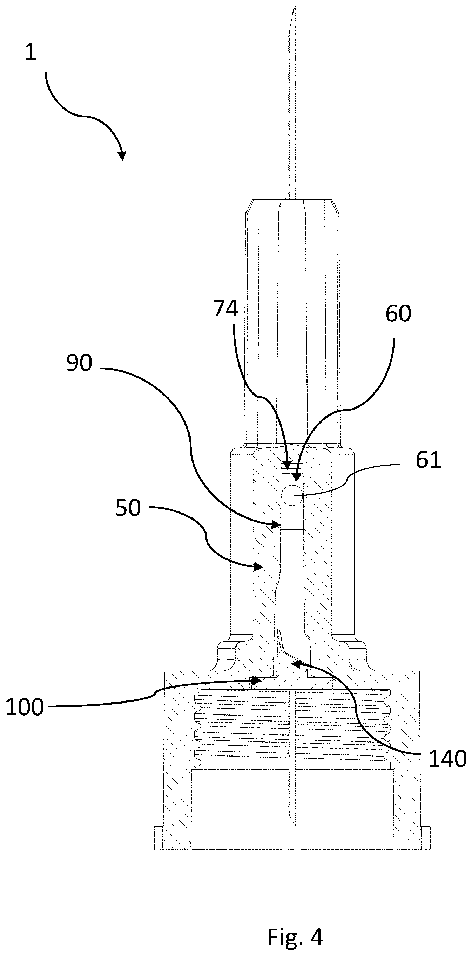

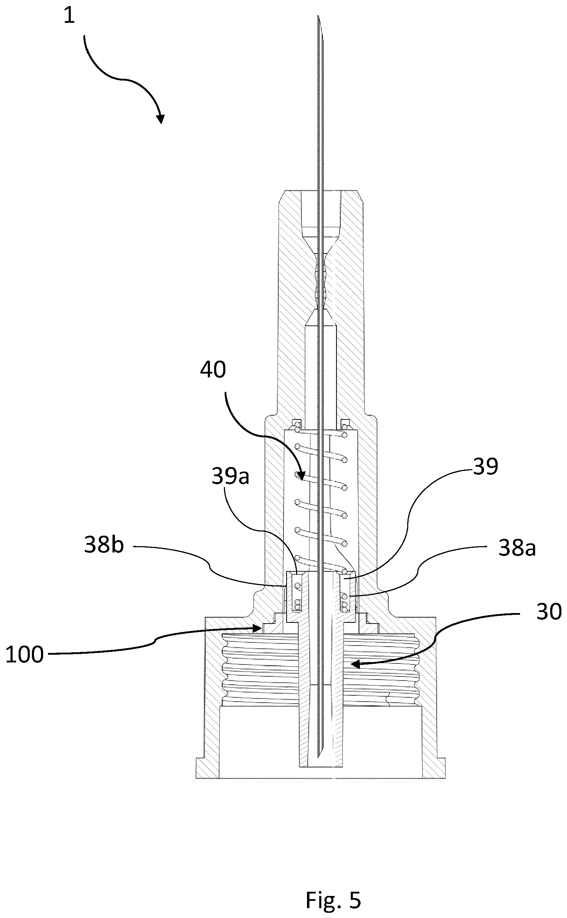

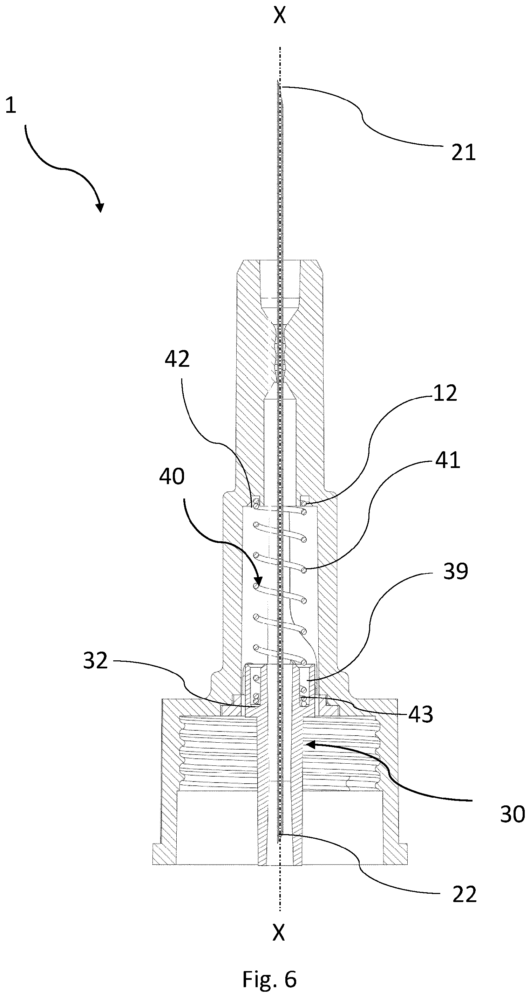

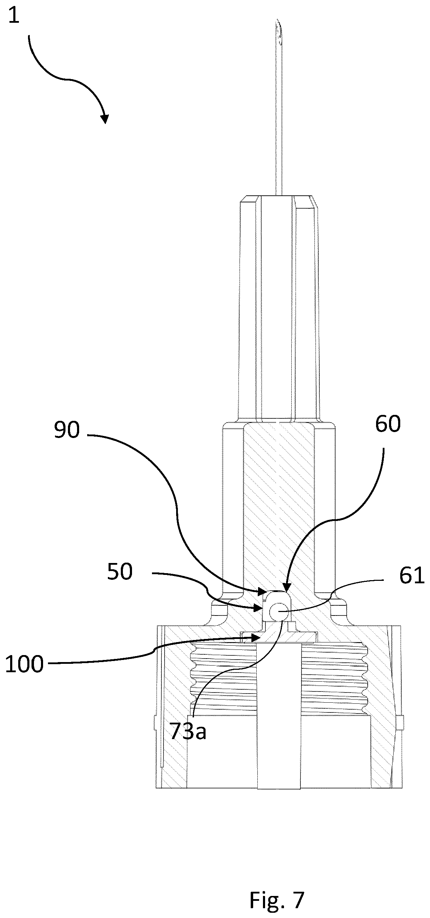

According to the first embodiment, the back-end shield 30, as shown in figures 1 and 2, in the initial position covers the proximal end 22 of the needle 20. Subsequently, the back-end shield 30, as shown in figures 3 and 4, in the intermediate position leaves uncovered a first portion 23 of the needle 20 arranged inside the cavity 13 and also the proximal end 22 of the needle 20. Finally, the back-end shield 30, as shown in figure 5, in the locking position covers the proximal end 22 of the needle 20. It is worth noting that upon reaching the locking position the back-end shield 30 is configured to cover the proximal end 22 of the needle 20. Optionally, in the locking position a limited sliding the back-end shield 30 along the longitudinal direction X-X is also allowed without uncovering the proximal end 22 of the needle 20 in figures 6 and 7 for example after removing the pen injector.

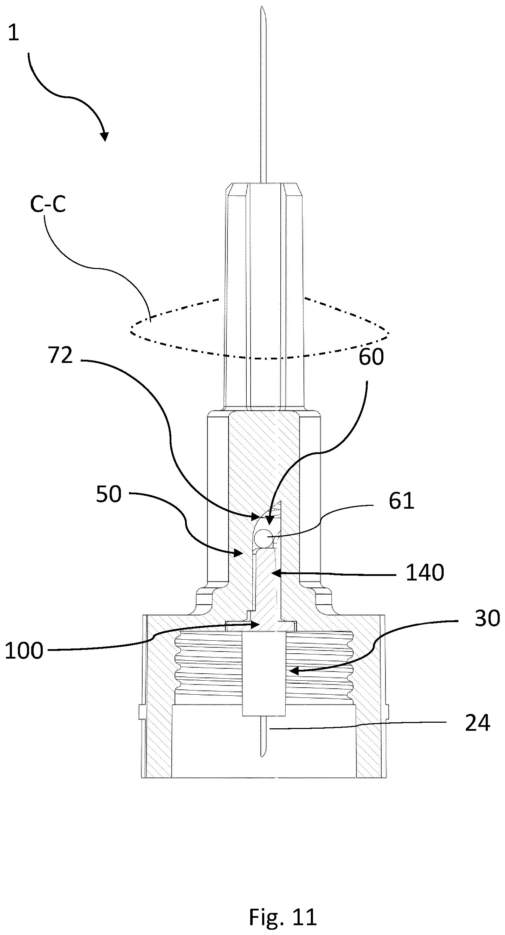

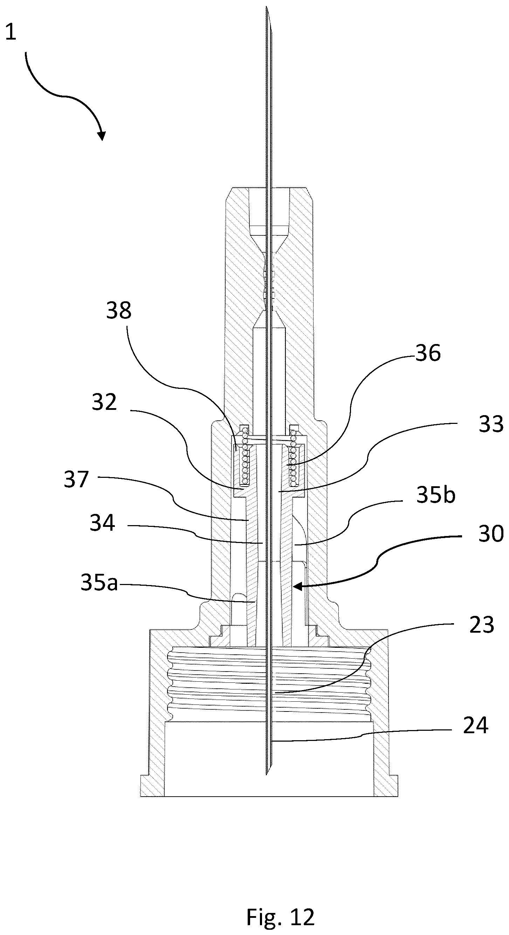

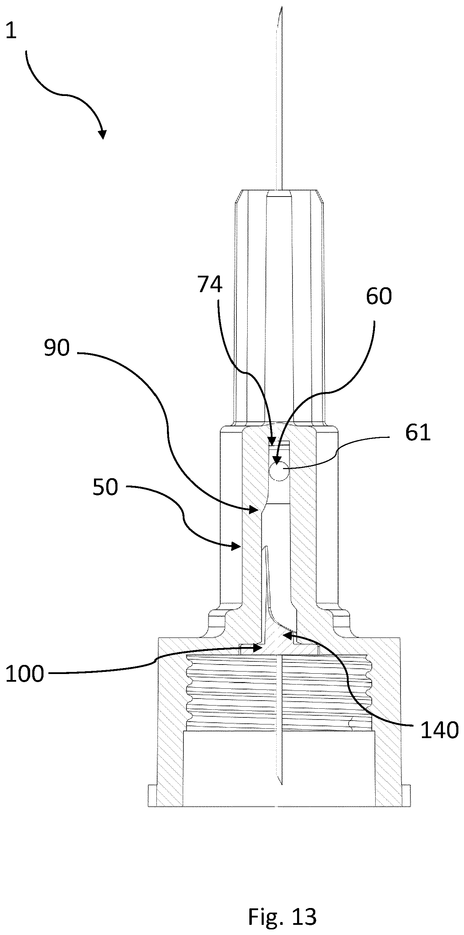

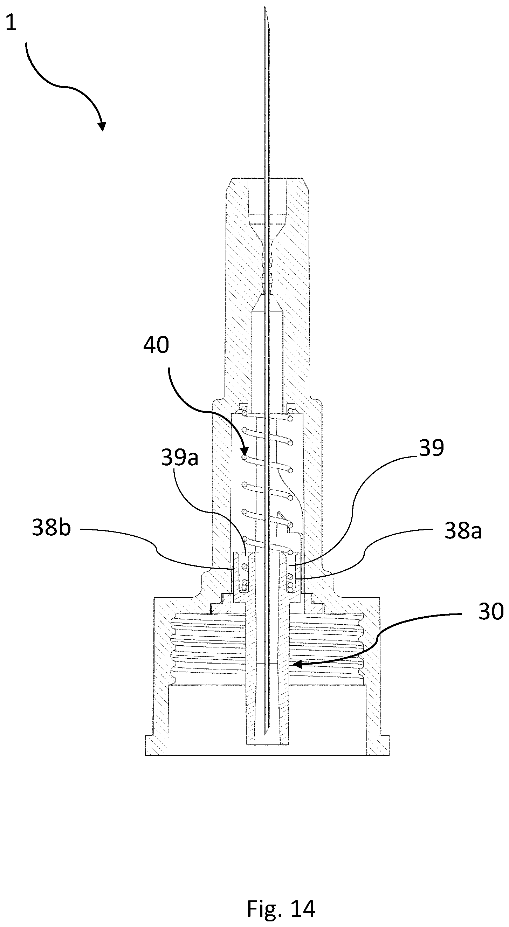

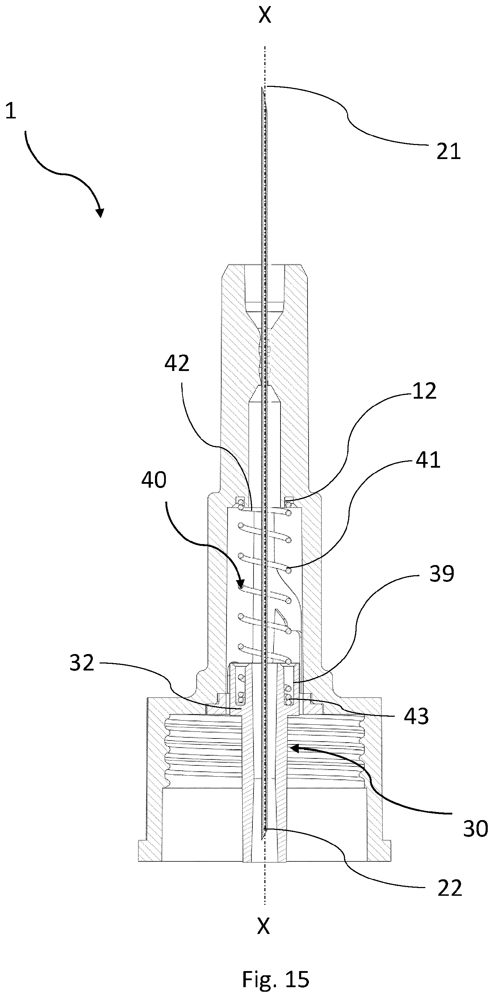

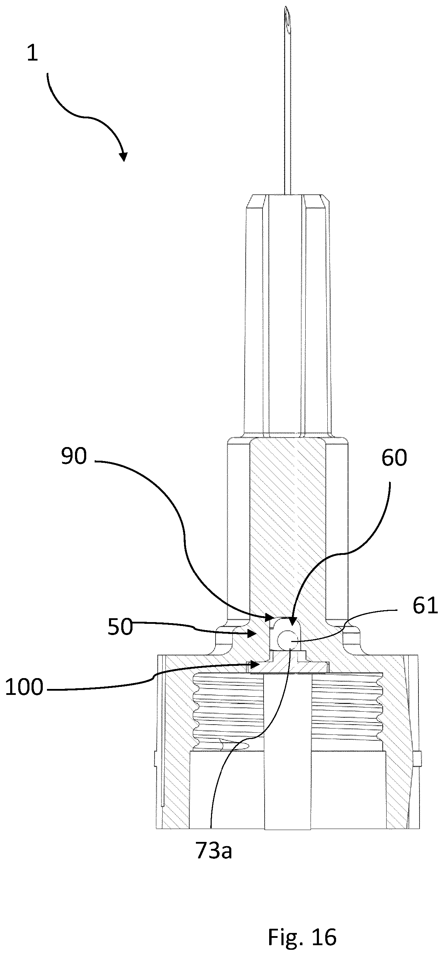

According to the second embodiment, the back-end shield 30, as shown in figure 10 and 11, in the initial position leaves uncovered a second portion 24 of needle 20 arranged inside the cavity 13 and the proximal end 22 of the needle 20. Subsequently, the back-end shield 30, as shown in figures 12 and 13, in the intermediate position leaves uncovered a first portion 23 of the needle 20 arranged inside the cavity 13 and also the proximal end 22 of the needle 20. It is to be noted that the second portion 24 being shorter than the first portion 23 because the back-end shield 30 has been moved towards the distal end 21 of the needle. Finally, the back-end shield 30, as shown in figure 14, in the locking position covers the proximal end 22 of the needle 20. It is worth noting that upon reaching the locking position the back-end shield 30 is configured to cover the proximal end 22 of the needle 20. Optionally, in the locking position a limited sliding the back-end shield 30 along the longitudinal direction X-X is also allowed without uncovering the proximal end 22 of the needle 20 in figures 15 and 16, for example after removing the pen injector.

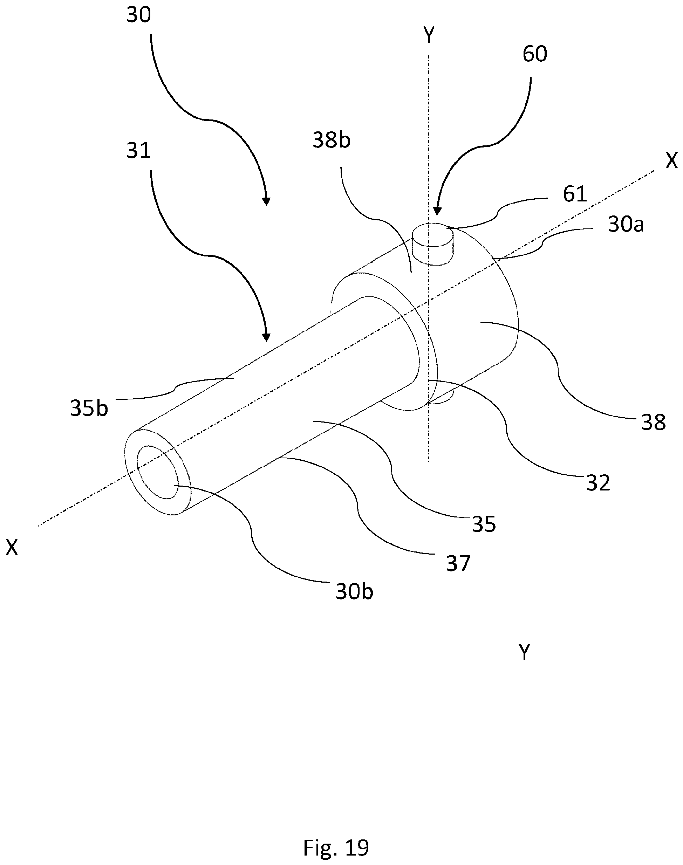

According to one embodiment, the back-end shield 30 (Figure 19) comprises an hallow body 31 extending between a distal portion 30a facing to the upper wall 12 of the hub 10 and a proximal portion 30b facing to the lower aperture 14 of the hub 10. Specifically, the back-end shield 30 is arranged inside the cavity 13. The hallow body 31 comprises a lower wall 32 formed proximal to the distal portion 30a. Such lower wall 32 has an opening 33 which is configured to allow a portion of the needle 20 to pass through while the back-end shield 30 is moving between the initial position and locking position. The hallow body 31 further comprises a lateral wall 35 extending from the lower wall 32 along the longitudinal direction X-X and defining a passing through channel 34. The passing through channel 34 is configured to house at least partially the needle 20 arranged inside the cavity 13. The lateral wall 35 has an inner surface 35a facing to the passing through channel 34 and an outer surface 35b facing to the cavity 13 of the hub 10.

According to one embodiment, the lateral wall 35 has a distal portion 36 extending along the longitudinal direction X-X from the lower wall 32 towards the distal end of the needle 20 and a proximal portion 37 extending along the longitudinal direction X-X from the lower wall 32 towards the proximal end of the needle 20. Preferably, the hallow body 31 comprises a retaining wall 38 extending along the longitudinal direction X-X from the lower wall 32 towards the distal end of the needle 20 and surrounding at least partially the lateral wall 35 preferably the distal portion 36 of lateral wall 35. Specifically, the lower wall 32, the retaining wall 38 and the distal portion 36 of the lateral wall 35 define the annular chamber 39 extending from the lower wall 32 to an annular aperture 39a. It is to be noted that, the retaining wall 38 has an inner surface 38a facing to the surrounded portion of the lateral wall 35, preferably facing to the distal portion 36 of the lateral wall 35, and an outer surface 38b facing to the cavity 13, preferably the inner surface of the hub 10.

The safety needle device 1 comprises an elastic member 40 arranged within the cavity 13 and acting between the upper wall 12 and the back-end shield 30 to urge the back-end shield 30 towards the proximal end 21 of the needle 10 along the longitudinal direction X-X. Preferably, the elastic member 40 is configured to constantly urge the back-end shield 30 towards the proximal end 22 of the needle 10. Specifically, the elastic member 40 generates a force directed to the proximal end 22 of the needle 20 as a result of a longitudinal motion of the back-end shield 30 towards the distal end 22 of the needle 20. In detail, the elastic member 40 is configured to be elastically compressed during the coupling of the pen injector with the hub 10, namely during the passage of the back-end shield 30 from the initial position to the intermediate position and to be elastically extended during the removal of the pen injector from the hub 10 with the passage of the back-end shield 30 from the intermediate position to the locking position. In other words, the pen injector acts on the hallow body 31 at the proximal portion 30b causing the motion of the back-end shield 30 from the initial position to the intermediate position and then the compression of the elastic member 40. After the removal of the pen injector and its action on the lower wall 32, the back-end shield 30 passes from the intermediate position to the locking position by means of elastic member 40 extension.

Preferably, the elastic member 40 is interposed between the hub 10 and the back-end shield 30 in order to constantly urge the back-end shield 30. Specifically, the elastic member 40 is at least partially arranged inside the annular chamber 39 which is configured to accommodate a portion of the elastic member 40. In details, the elastic member 40 is interposed between the upper wall 12 and the lower wall 32 in order to constantly urge the back-end shield 30 towards the proximal end 22.

It is to be noted that, the elastic member 40 surrounds at least partially the needle 20 passing inside the cavity 13 of the hub 10.

According to a preferred embodiment, the elastic member 40 comprises a spring member 41 which extends between a distal portion 42 and a proximal portion 43 along the longitudinal axis X-X. Accordingly, the portion 42 abuts against the upper wall 12 and the proximal portion 43 abuts against the lower wall 32.

The safety needle device 1 comprises a locking arrangement 50 configured to guide the back-end shied 30 from the initial position to the locking position and to lock the back-end shield 30 in the locking position. Preferably, the locking arrangement 50 is configured to rotate the back-end shield 30 upon sliding of the back-end shield 30 along the longitudinal direction X-X and to lock the back-end shield 30 in order to constantly cover the proximal end 22 of the needle 20.

The locking arrangement 50 comprises at least a first locking member 60 formed on the back-end shield 30 and at least a second locking member 70. Specifically, the second locking member 70 cooperates with the first locking member 60 to guide the back-end shield 30 from the initial position to the locking position and to lock the back-end shield 30 in the locking position.

The second locking member 70 comprises a first guide element 90 formed on the lateral wall 11 of the hub and a second guide element 100 attached to the lateral wall 11 inside the cavity 13 at the intermediate aperture 15. Specifically, the first guide element 90 and the second guide element 100 define a guide track 71 inside the cavity 13. The first locking member 60 comprises a protrusion 61 configured to engage the first guide element 90 and the second guide element 100 to slide along the guide track 71.

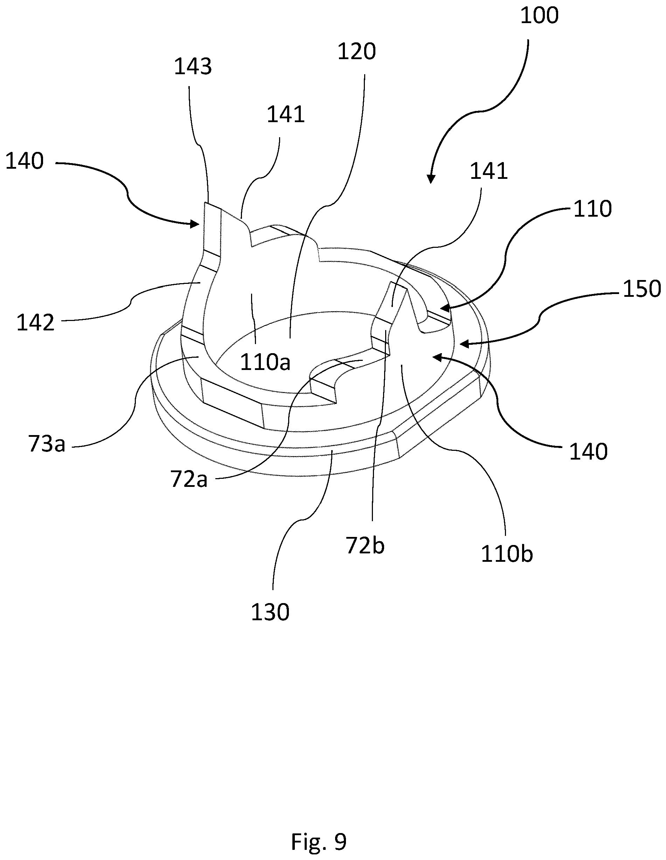

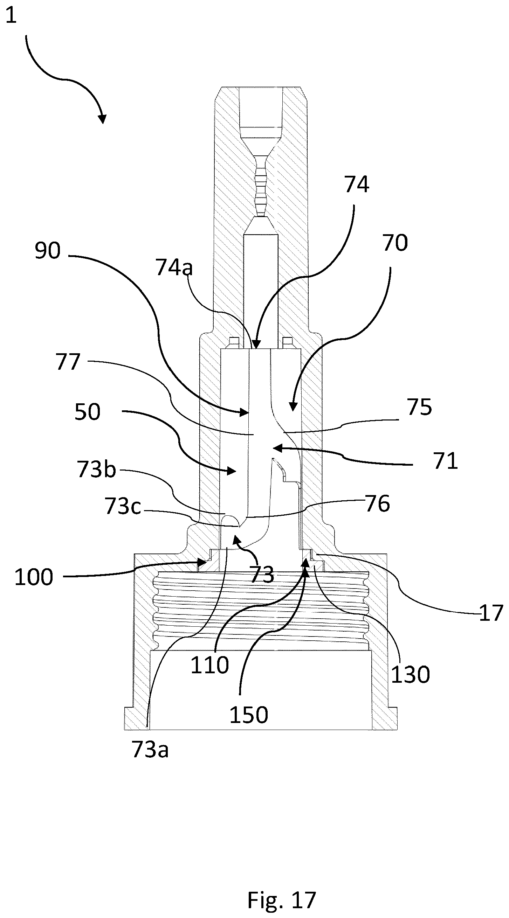

It is to be noted that the guide track 7171 has a shape shown in figures 9 and 17.

According to a preferred embodiment, the protrusion 61 is formed on the outer surface 38b of the retaining wall 38 of the back-end shield 30, preferably projecting from the outer surface 38b along a transversal direction Y-Y perpendicular to the outer surface 38b and then to the longitudinal direction X-X. Preferably, the protrusion 61 is formed proximal to the distal portion 30a of the back-end shield 30. More preferably, the locking arrangement 50 comprises at least two protrusions 61 mutually spaced apart along the outer surface 38b of the retaining wall 38. Even more preferably, the two protrusions 61 are circumferentially spaced apart on the back-shield 30. The preferred embodiment shown in the figures, the protrusions 61 are symmetric and circumferentially spaced apart on the outer surface 35b of the back-end shield.

According to a preferred embodiment, the protrusions 61 are pins.

According to one embodiment, the first guide element 90 is formed on the lateral wall 11 of the hub 10. Preferably, the first guide element 90 comprises a groove formed on the inner surface 10a of the hub 10. More preferably, the hub 10 comprises two symmetric first guide elements 90 circumferentially spaced apart on the lateral wall 11. In other words, the hub 10 has two first guide elements 90 angularly spaced apart along the lateral wall 11.

Such configuration cooperating with the second guiding element 100 allows to correctly guide the back-end shield 30 between the initial position to the locking position and cooperates with the protrusions to lock the back-end shield 30 in the locking position.

According to one embodiment, the second guide element 100 comprises an annular portion 110 configured to engage the lateral wall 11 at the intermediate aperture 15. Specifically, the annular portion 110 defines a passing through channel 120 to allow the sliding of the back-end shield 30 along the longitudinal direction X-X. Preferably, the annular portion 110 comprises an inner surface 100a facing the back-end shield 30 and an outer surface 100b facing the lateral wall 11 of the hub 10. The annular portion 100 has flange 130 protruding from the outer surface 100b and configured to engage the lateral wall 11 of the hub.

According to one embodiment, the annular portion 110 has retaining means 150 configured to engage with the lateral wall 12 at the intermediate aperture 15. Specifically, the hub 10 comprises at intermediate aperture 15 a retaining groove 17 formed on the lateral wall 12. The retaining groove 17 has a circumferential profile and facing the lower aperture 13. The retaining means 150 are configured to engage the retaining groove 17 to attach the second guiding element 100 to the hub 10. Preferably, the retaining means 150 allows to engage the hub 10 by interference fit. More preferably, the annular portion 110 and the flange 130 are configured to be inserted into the retaining groove 17 and be attached to the hub by interference fit. It is to be noted that the retaining means 150 allow to remove the annular portion 110 from the hub 10.

The second guide element 100 further comprises at least a guiding wing 140, preferably two, projecting from the annular portion 110 towards the distal end 21 inside the cavity 13 and configured to engage the protrusion 61 and guide it from initial position to the locking position. Preferably, guiding wings 140 are angularly spaced apart along the annular portion 110. In other words, the guiding wings 140 are circumferentially spaced apart on the annular portion 110. It is to be noted that the guiding wing 140 follow a circumferential profile defined by the annular portion 110. In this way, the guiding wing 140 extends the passing through channel 120 in order to allow the sliding of the back-end shield 30 along the longitudinal direction X-X.

It is to be noted that upon attachment of the annular portion 110 to the hub the guiding wing 140 are inserted into the cavity 13 facing the upper wall 12.

According to one embodiment, the first guiding element 90 and the second guiding element 100 defines a first stopping area 72 and a second stopping area 73.

The first stopping area 72 is configured to engage the protrusion 61 in the initial position of the back-end shield 30 to prevent longitudinal sliding of the back-end shield 30 towards the proximal end 22 of the needle 20. Preferably, the first stopping area 72 has a first support portion 72a that retains the protrusion 61 and maintains the back-end shield 30 in the initial position and a second support portion 72b that prevents undesired rotation of the back-end shield 30 about the longitudinal direction X-X. More preferably the second support portion 72b extends along the longitudinal direction X-X from the first support portion 72a towards the distal end 22 of the needle 20. Even more preferably, the first support portion 72a and the second support portion 72b define an initial bay which retains the back-end shield 30 in the initial position until the coupling of the pen injector with the hub 10. As a matter of fact, the first stopping area 72 retains the back-end shield 30 against preloaded elastic force of the elastic member 40 which urges the back-end shield 30 towards the proximal end 22.

According to a preferred embodiment shown in the figures, the annular portion 110 defines the first support portion 72a and the guiding wing 140 defines at least partially the second support portion 72b.

The second stopping area 73 is configured to engage the protrusion 61 in the locking position of the back-end shield 30 to prevent longitudinal sliding of the back-end shield 30 both towards the distal end 21 and the proximal end 22 of the needle 10. Specifically, the second stopping area 73 is configured to retain the protrusion 61 in order to prevent further use of the safety needle device 1 and the sliding of the back-end shield along the longitudinal direction X-X. Preferably, the second stopping area 73 has a first support portion 73a and a second support portion 73b which prevent the longitudinal sliding of the back-end shield 30 both towards the distal end 21 and the proximal end 22 of the needle. In detail, also the first support portion 73a limits the stroke of the back-end shield 30 along the longitudinal axis X-X retaining the shield 30 against preloaded elastic force of the elastic member 40 which continues to urge the back-end shield 30 towards the proximal end 21 also in the locking position. More preferably, the second stopping area 73 has a third support portion 73c which prevent rotation of the back-end shield 30 about the longitudinal direction X-X. Specifically, the third support portion 73c extends from the second support potion 73b along the longitudinal direction X-X towards the proximal end 21 of the needle 20. Even more preferably, the support portion 73a, 73b, 73c of the second stopping member 72 defines a locking bay which engages and retains the protrusion 61 when the back-end shield 30 reaches the locking position. In this way the back-end shield is retained in the locking position and the proximal end 22 of the needle is covered after use of the safety needle device.

According to a preferred shown in the figures, the annular portion 110 comprises the first support portion 73a and the first guiding element 90 comprises the second support portion 73b and third support portion 73c.

In other words, the first stopping area 72 and the second stopping area 73 have substantially a "C" shape wherein the "C" of the first stopping is rotated ninety degrees counterclockwise.

It is to be noted that, the first stopping area 72 and second stopping area 73 are circumferentially spaced apart. More preferably, the first stopping area 72 and second stopping area 73 are spaced apart along a circumferential direction C-C defined around the hub 10

According to the preferred embodiment shown in figures 1-9, the first stopping area 72 and the second stopping area 73 are arranged proximal to the intermediate aperture 15. Specifically, the first stopping area 72 and the second stopping area 73 are substantially on a same plane perpendicular to the longitudinal direction X-X. Accordingly, as reported above, in the initial position the back-end shield 30 covers the proximal end 22 of the needle 20. In detail, first support portion 72a of the first stopping area 72 and the first support portion 73a of the second stopping area 73 are on the same plane.

According to the preferred embodiment shown in figures 10-18, alternative to the previous embodiment, the first stopping area 72 is arranged towards the distal end 21 of the needle 20 and the second stopping area 73 is arranged towards the proximal end 22 of the needle 20. Specifically, the first stopping area 72 and the second stopping area 73 are offset along the longitudinal direction X-X. In other words, the first stopping area 72 and the second stooping area 73 are mutually spaced apart along the longitudinal direction X-X, with the first stopping area 72 towards the distal end of the needle and the second stooping area 73 towards the proximal end of the needle.

In other words, first support portion 72a of the first stopping area 72 72 is arranged towards the distal end 21 of the needle 20, while the first support portion 73a of the second stopping area 73 arranged towards the proximal end 22 of the needle 20.

According to a preferred embodiment, the first guiding element 90 comprises a third stopping area 74 configured to engage the protrusion 61 in the intermediate position to prevent longitudinal sliding of the back-end shield 30 towards the distal end 22 of the needle 20. Preferably, the third stopping area 74 limits the stroke of the protrusion along the longitudinal direction X-X. More preferably, the first guiding element 90 comprises at the third stopping area 74 a support portion 74a configured to engage the protrusion 61 in the intermediate position to prevent longitudinal sliding of the back-end shield 30 towards the distal end 22 of the needle 20. Specifically, the protrusion 61 abuts against the support portion 74a of the third stopping member 74 in the intermediate position.

It is to be noted that the third stopping area 74 is spaced apart from the first stopping area 72 and from the second stopping area 73 along the longitudinal direction X-X towards the distal end 21 of the needle 20. At the same time, the third stopping area 74 is circumferentially spaced apart both from the first stopping area 72 and the second stopping member 73. Preferably, the third stopping area 74 is arranged in the middle between the first stopping area 72 and the second stopping area 73 along the circumferential direction C-C which goes around the hub 10.

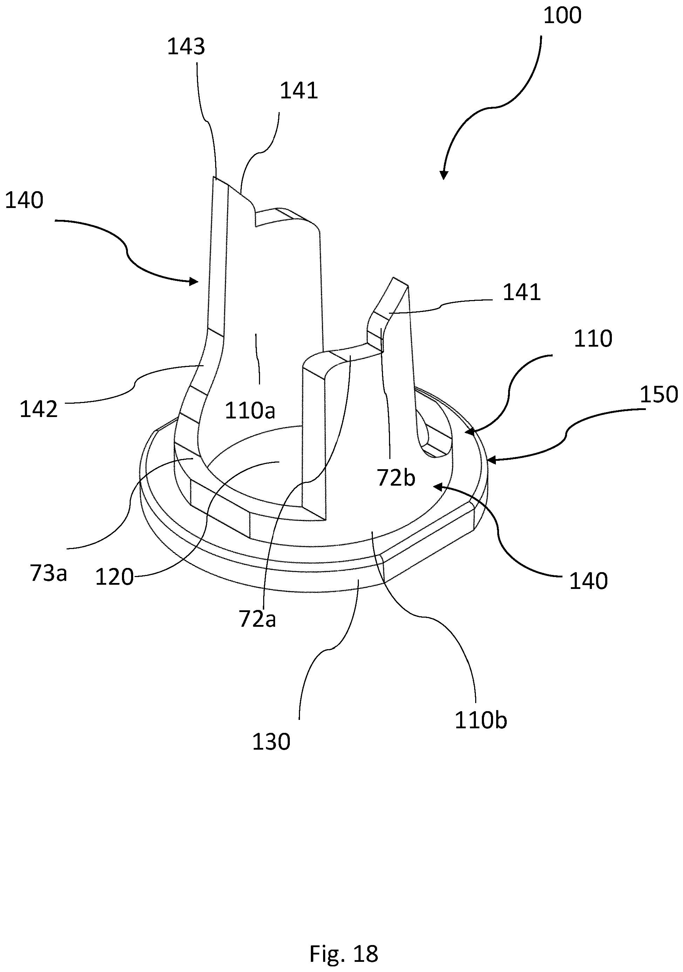

According to one embodiment, the guiding wing 140 comprises a first wing rotating portion 141 and a second wing rotating portion 142 configured to rotate the back-end shield 30 upon sliding along the longitudinal direction X-X cooperating with the first guiding element 90. Specifically, the first wing rotating portion 141 and a second wing rotating portion 142 (figures 9 and 18) extends from the annular portion 110 along inclined direction with respect to the longitudinal direction X-X up to a connecting apex 143. In other words, the guiding wing 140 is tapered from the annular portion 110 towards the distal end along longitudinal direction X-X up to a connecting apex 143 defining on the opposed slopes the first wing rotating portion 141 and the second wing rotating portion 142.

The first wing rotating portion 141 is configured to guide and to rotate the back-end shield 30 upon sliding of the back-end shield 30 along the longitudinal axis X-X from the initial position towards the intermediate position. Preferably, the first wing rotating portion 141 is configured to engage the protrusion 61 and cooperating with the first guiding element 90 to rotate the back-end shield 30 upon sliding of back-end shield 30 along the longitudinal axis X-X from the initial position towards the intermediate position.
Instead, the second wing rotating portion 142 is configured to guide and rotate the back-end shield 30 upon sliding of the back-end shield 30 along the longitudinal axis X-X from the intermediate position towards the locking position. Preferably, the second wing rotating portion 142 is configured to engage the protrusion 61 and cooperating with the first guiding element 90 to rotate the back-end shield 30 upon sliding of back-end shield 30 along the longitudinal axis X-X from the intermediate position towards the locking position. More preferably, the second wing rotating portion 142 is arcuate and allows the rotation of the back-end shield 30 during the sliding along the longitudinal direction X-X. The combination of the rotation and the sliding allows the protrusion to engage the second stopping area 73.

According to a preferred embodiment, the first guiding element 90 comprises a first rotation portion 75 and a second rotation portion 76 configured to rotate the back-end shield 30 upon sliding along the longitudinal direction X-X cooperating with the second guiding element 100. Preferably, the first rotation portion 75 cooperates with the first wing rotating portion 141 and the second rotation portion 76 cooperates with the second wing rotating portion 142.

Specifically, the first rotation portion 75 is configured to engage the protrusion 61 and cooperating with the second guiding element 100, preferably with the first wing rotating portion 141, to rotate the back-end shield 30 upon sliding of back-end shield 30 along the longitudinal axis X-X from the initial position towards the intermediate position. Preferably the first rotation portion 75 is arcuate and allows the rotation of the back-end shield 30 during the sliding along the longitudinal direction X-X. The combination of the rotation and the sliding allows the protrusion 61 to get out from the first stopping area 72.

The second rotation portion 76 is configured to engage the protrusion 61 and cooperating with the second guiding element 100, preferably with the second wing rotating portion 142, to rotate the back-end shield 30 upon sliding of the back-end shield 30 along the longitudinal axis X-X from the intermediate position towards the locking position. Preferably, the second rotation portion 76 drives the protrusion 61 to the second wing rotating portion 142 allowing the rotation of the back-end shield 30 during the sliding along the longitudinal direction X-X. The combination of the rotation and the sliding allows the protrusion 61 to engage the second stopping area 73.

According to a preferred embodiment, the first guiding element 90 comprises a linear guiding portion 77 configured to engage the protrusion 61 and guide the back-end shield 30 along longitudinal axis X-X preventing rotation of the back-end shield 30 from the initial position towards the intermediate position and from the intermediate position towards the locking position. Preferably, the linear guiding portion 77 connects the first stopping area 72 and the second stopping area 73 with the third stopping area 74 such that to guide the protrusion 61 during the passage of the back-end shield 30 from the initial position to intermediate position and then from the intermediate position to the final position.

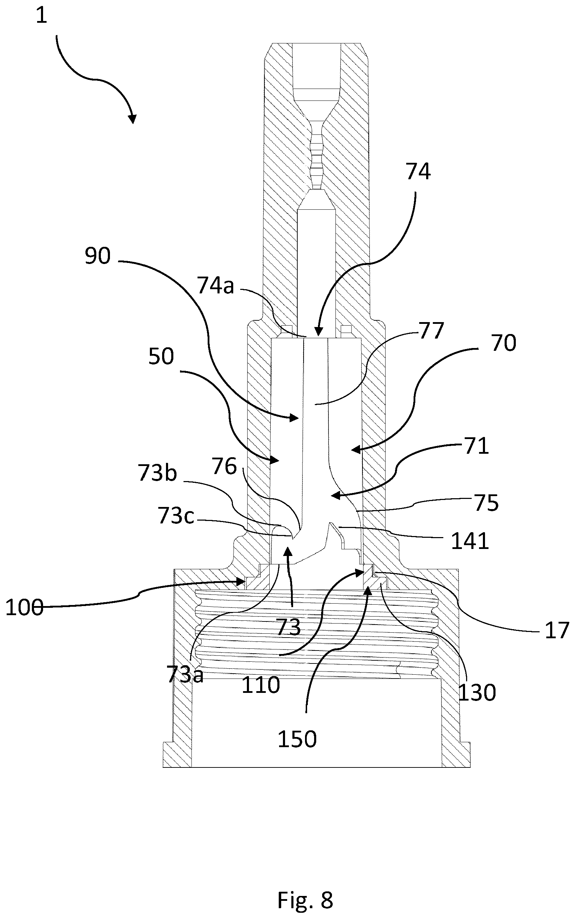

According to the preferred embodiments as shown in figures 8 and 17, the first guiding element 90 and the second guiding element 100 define a guide track 71. Specifically, the guide track 71 extends from the first stopping area 72 defined by the combination of the annular portion 110 and a portion of the first guiding element 90, preferably the first rotation portion 75. Then, the guide track 71 has the linear guiding portion 77 which is connected to the first stopping area 72 by the first rotation portion 75 and the first wing rotating portion 141. In detail, the first rotation portion 75 and the first wing rotating portion 141 route the protrusion 61, rotating the back-end shield 30, from the first stopping area 72 to the linear guiding portion 77. The latter extends up to the third stopping area 73 wherein the protrusion 61 abuts in the intermediate position. The guide track 71 drives the protrusion 61 from the intermediate position to the locking position along the linear guiding portion 77 towards the proximal end of the needle. Preferably, the protrusion 61 slides along the linear guiding portion 77 two times in opposed verses along the longitudinal direction X-X:
- towards the distal end of the needle during the sliding from the initial position to the intermediate position;
- towards the proximal end of needle during the sliding from the intermediate position to the locking position.

The guide track 71 has the second rotation portion 77 and the second wing rotating portion 142 which deviates the protrusion from it linear sliding along the longitudinal direction X-X towards the proximal end of the needle to the second stopping area 73. Specifically, second rotation portion 76 and the second wing rotating portion 146 route the protrusion 61, rotating the back-end shield 30 from the linear guiding portion 77 to the second stopping area 73. Finally, the guide track 71 has the stopping area 73 wherein the protrusion 61 is locked after use.
According to one embodiment, the hub 10 comprises two symmetric guides tracks 71 circumferentially spaced apart on the lateral wall 11, one for each protrusion 61. Thanks to the symmetry the sliding and the rotating of the back-end shield is improved avoiding locks during the sliding.

## Claims

1. Safety needle device (1) comprising:
- a hub (10) configured to be connected to a pen-injector, the hub (10) comprising a lateral wall (11) and an upper wall (12) which define therebetween a cavity (13) extending between the upper wall (12) and a lower aperture (14) able to receive the pen injector;
- a needle (20) attached to the hub (10), the needle (20) extending along a longitudinal direction (X-X) between a distal end (21), configured for insertion into a patient, and an opposed proximal end (22) configured to be connected to the pen injector and arranged inside the cavity (13),
- a back-end shield (30) slidable along the longitudinal direction (X-X) within the cavity (13) of the hub (10) and rotatable about the longitudinal direction (X-X), the back-end shield (30) being movable irreversibly from an initial position, before connection of the pen injector to the hub (10), to a locking position, after removal of the pen injector to the hub (10) passing through an intermediate position,
- an elastic member (40) arranged within the cavity (13) and acting between the upper wall (12) and the back-end shield (30) to urge the back-end shield (30) towards the proximal end (21) of the needle (10) along the longitudinal direction (X-X),
- a locking arrangement (50) comprising at least a first locking member (60) formed on the back-end shield (30) and at least a second locking member (70), the second locking member (70) cooperating with the first locking member (60) to guide the back-end shield (30) from the initial position to the locking position and to lock the back-end shield (30) in the locking position,
wherein
- the second locking member (70) comprises a first guide element (90) formed on the lateral wall (11) of the hub (10) and a second guide element (100) attached to the lateral wall (11) inside the cavity (13) at an intermediate aperture (15) defined between the upper wall (12) and the lower aperture (14), the first guide element (90) and the second guide element (100) defining a guide track (71) inside the cavity (13);
- the first locking member (60) comprises a protrusion (61) configured to engage the first guide element (90) and the second guide element (100) to slide along the guide track (71).

2. Safety needle device (1) according to claim 1, wherein the second guide element (100) comprises:
- an annular portion (110) configured to engage the lateral wall (11) at the intermediate aperture (15) and defining a passing through channel (120) to allow the sliding of the back-end shield (30) along the longitudinal direction (X-X);
- a guiding wing (140) projecting from the annular portion (110) towards the distal end (21) inside the cavity (13) and configured to engage the protrusion (61) and guide it from initial position to the locking position.

3. Safety needle device (1) according to claim 2, wherein the guiding wing (140) comprises:
- a first wing rotating portion (141) configured to cooperate with the first guiding element (90) to guide and to rotate the back-end shield (30) upon sliding of the back-end shield (30) along the longitudinal axis (X-X) from the initial position towards the intermediate position;
- a second wing rotating portion (142) configured to cooperate with the first guiding element (90) to guide and rotate the back-end shield (30) upon sliding of the back-end shield (30) along the longitudinal axis (X-X) from the intermediate position towards the locking position.

4. Safety needle device (1) according to claim 2 or 3, wherein the annular portion (110) has retaining means (150) configured to engage with the lateral wall (12) at the intermediate aperture (15).

5. Safety needle device (1) according to any claims 1 to 4, wherein the first guiding element (90) and the second guiding element (100) define:
- a first stopping area (72) configured to engage the protrusion (61) in the initial position of the back-end shield (30) to prevent longitudinal sliding of the back-end shield (30) towards the proximal end (22) of the needle (20);
- a second stopping area (73) configured to engage the protrusion (61) in the locking position of the back-end shield (30) to prevent longitudinal sliding of the back-end shield (30) both towards the distal end (21) and the proximal end (22) of the needle (10).

6. Safety needle device (1) according to claim 5, wherein:
- the first stopping area (72) and the second stopping area (73) are arranged proximal to the intermediate aperture (15);
- the back-end shield (30) in the initial position covers the proximal end (22) of the needle (20);
- the back-end shield (30) in the intermediate position leaves uncovered a first portion (23) of the needle (20) arranged inside the cavity (13) and the proximal end (22) of the needle (20);
- the back-end shield (30) in the locking position covers the proximal end (22) of the needle (20).

7. Safety needle device (1) according to claim 5, wherein:
- the first stopping area (72) is arranged towards the distal end (21) of the needle (20) and the second stopping area (73) is arranged towards the proximal end (22) of the needle (20);
- the back-end shield (30) in the initial position leaves uncovered a second portion (24) of needle (20) arranged inside the cavity (13) and the proximal end (22) of the needle (20);
- the back-end shield (30) in the intermediate position leaves uncovered a first portion (23) of the needle (20) arranged inside the cavity (13) and the proximal end (22) of the needle (20), the second portion (24) being shorter than the first portion (23)
- the back-end shield (30) in the locking position covers the proximal end (22) of the needle (20).

8. Safety needle device (1) according to claim any claims 4 to 7, wherein first guiding element (90) defines:
- a third stopping area (74) configured to engage the protrusion (61) in the intermediate position to prevent longitudinal sliding of the back-end shield (30) towards the distal end (22) of the needle (20);
- the third stopping area (74) being spaced apart from the first stopping area (72) and the second stopping area (73) along the longitudinal direction (X-X) towards the distal end (21) of the needle (20).

9. Safety needle device (1) according to any claims 1 to 8, wherein the first guiding element (90) comprises:
- a first rotation portion (75) configured to engage the protrusion (61) and cooperating with the second guiding element (100) to rotate the back-end shield (30) upon sliding of back-end shield (30) along the longitudinal axis (X-X) from the initial position towards the intermediate position;
- a second rotation portion (76) configured to engage the protrusion (61) and cooperating with the second guiding element (100) to rotate the back-end shield (30) upon sliding of the back-end shield (30) along the longitudinal axis (X-X) from the intermediate position towards the locking position.

10. Safety needle device (1) according to any claims 1 to 9, wherein the first guiding element (90) comprises:
- a linear guiding portion (77) configured to engage the protrusion (61) and guide the back-end shield (30) along longitudinal axis (X-X) preventing rotation of the back-end shield (30) from the initial position towards the intermediate position and from the intermediate position towards the locking position.

11. Safety needle device (1) according to claim any claims 1 to 9, wherein:
- the hub (10) has an inner surface (10a) facing to the cavity (13) and opposed outer surface (10b);
- the first guide element (90) comprises a groove formed on the inner surface (10a);
- the protrusions (61) are pins.
- the elastic member (40) comprises a spring element (41) extending between a distal portion (42) and a proximal portion (43) along the longitudinal axis (X-X).

12. Safety needle device (1) according to any of claims 1 to 11, wherein the back-end shield (30) comprises an hallow body (31) extending between a distal portion (30a) facing to the upper wall (12) of the hub (10) and a proximal portion (30b) facing to the lower aperture (14) of the hub (10), the hallow body (31) comprising:
- a lower wall (32) formed proximal to the distal portion (30a) and having an opening (33) configured to allow a portion of the needle (20) and the proximal end (22) of the needle (20) to pass upon moving of the back-end shield (30) between the initial position and locking position;
- a lateral wall (35) extending from the lower wall (32) along the longitudinal axis (X-X) and defining a passing through channel (34) configured to house at least partially the needle (20);
- a retaining wall (38) extending along the longitudinal direction X-X from the lower wall (32) towards the distal end of the needle (20) and surrounding a portion of the lateral wall (35).

13. Safety needle device (1) according to claim 12, wherein the retaining wall (38) has an inner surface (38a) facing the surrounded portion of the lateral wall 35 and an outer surface (38b) facing to the cavity (13) of the hub (10) and the protrusions (61) are formed on the outer surface (38b) of the back-end shield (30).

14. Safety needle device (1) according to claim 12 or 13, wherein:
- the elastic member (40) is interposed between the upper wall (12) of the hub (10) and the lower wall (32) of the back-end shield (30);
- the elastic member (40) surrounds at least partially the needle (20) passing inside the cavity (13) of the hub (10).

15. Safety needle device (1) according to any claims 1 to 14, wherein
- the hub (10) comprises two symmetric guides tracks (71) circumferentially spaced apart on the lateral wall (11);
- the back-end shield (30) comprises two symmetric protrusions (61) circumferentially spaced apart on the back-end shield (30),
- the second guiding element (100) comprises two symmetric guiding wings (140) circumferentially spaced apart on the annular portion (110).

## Patentansprüche

1. Sicherheitsnadelvorrichtung (1), umfassend:
- eine Nabe (10), die dazu konfiguriert ist, mit einem Pen-Injektor verbunden zu werden, wobei die Nabe (10) eine Seitenwand (11) und eine obere Wand (12) umfasst, die zwischen sich einen Hohlraum (13) definieren, der sich zwischen der oberen Wand (12) und einer unteren Öffnung (14) erstreckt, die in der Lage ist, den Pen-Injektor aufzunehmen;
- eine an der Nabe (10) angebrachte Nadel (20), wobei sich die Nadel (20) entlang einer Längsrichtung (X-X) zwischen einem distalen Ende (21), das zur Einführung in einen Patienten konfiguriert ist, und einem gegenüberliegenden proximalen Ende (22) erstreckt, das zur Verbindung mit dem Pen-Injektor konfiguriert und innerhalb des Hohlraums (13) angeordnet ist;
- einen hinteren Schutzschild (30), der entlang der Längsrichtung (X-X) innerhalb des Hohlraums (13) der Nabe (10) verschiebbar und um die Längsrichtung (X-X) drehbar ist, wobei der hintere Schutzschild (30) irreversibel aus einer Ausgangsposition, vor dem Verbinden des Pen-Injektors mit der Nabe (10), in eine Verriegelungsposition, nach dem Entfernen des Pen-Injektors von der Nabe (10), bewegbar ist, wobei er eine Zwischenposition durchläuft;
- ein elastisches Element (40), das innerhalb des Hohlraums (13) angeordnet ist und zwischen der oberen Wand (12) und dem hinteren Schutzschild (30) wirkt, um den hinteren Schutzschild (30) entlang der Längsrichtung (X-X) in Richtung des proximalen Endes (21) der Nadel (10) zu drängen;
- eine Verriegelungsanordnung (50), die mindestens ein erstes Verriegelungselement (60), das am hinteren Schutzschild (30) ausgebildet ist, und mindestens ein zweites Verriegelungselement (70) umfasst, wobei das zweite Verriegelungselement (70) mit dem ersten Verriegelungselement (60) zusammenwirkt, um den hinteren Schutzschild (30) von der Ausgangsposition in die Verriegelungsposition zu führen und den hinteren Schutzschild (30) in der Verriegelungsposition zu verriegeln,
wobei
- das zweite Verriegelungselement (70) ein erstes Führungselement (90), das an der Seitenwand (11) der Nabe (10) ausgebildet ist, und ein zweites Führungselement (100) umfasst, das an der Seitenwand (11) innerhalb des Hohlraums (13) an einer Zwischenöffnung (15) angebracht ist, die zwischen der oberen Wand (12) und der unteren Öffnung (14) definiert ist, wobei das erste Führungselement (90) und das zweite Führungselement (100) eine Führungsbahn (71) innerhalb des Hohlraums (13) definieren;
- das erste Verriegelungselement (60) einen Vorsprung (61) umfasst, der dazu konfiguriert ist, mit dem ersten Führungselement (90) und dem zweiten Führungselement (100)in Eingriff zu treten, um entlang der Führungsbahn (71) zu gleiten.

2. Sicherheitsnadelvorrichtung (1) nach Anspruch 1, wobei das zweite Führungselement (100) umfasst:
- einen ringförmigen Abschnitt (110), der dazu konfiguriert ist, mit der Seitenwand (11) an der Zwischenöffnung (15) in Eingriff zu treten und einen Durchgangskanal (120) zu definieren, um das Gleiten des hinteren Schutzschilds (30) entlang der Längsrichtung (X-X) zu ermöglichen;
- einen Führungsflügel (140), der von dem ringförmigen Abschnitt (110) in Richtung des distalen Endes (21) innerhalb des Hohlraums (13) vorsteht und dazu konfiguriert ist, mit dem Vorsprung (61) in Eingriff zu treten und ihn von der Ausgangsposition in die Verriegelungsposition zu führen.

3. Sicherheitsnadelvorrichtung (1) nach Anspruch 2, wobei der Führungsflügel (140) umfasst:
- einen ersten Flügel-Drehabschnitt (141), der dazu konfiguriert ist, mit dem ersten Führungselement (90) zusammenzuwirken, um den hinteren Schutzschild (30) beim Gleiten des hinteren Schutzschilds (30) entlang der Längsachse (X-X) von der Ausgangsposition in Richtung der Zwischenposition zu führen und zu drehen;
- einen zweiten Flügel-Drehabschnitt (142), der dazu konfiguriert ist, mit dem ersten Führungselement (90) zusammenzuwirken, um den hinteren Schutzschild (30) beim Gleiten des hinteren Schutzschilds (30) entlang der Längsachse (X-X) von der Zwischenposition in Richtung der Verriegelungsposition zu führen und zu drehen.

4. Sicherheitsnadelvorrichtung (1) nach Anspruch 2 oder 3, wobei der ringförmige Abschnitt (110) Haltemittel (150) aufweist, die dazu konfiguriert sind, mit der Seitenwand (12) an der Zwischenöffnung (15) in Eingriff zu treten.

5. Sicherheitsnadelvorrichtung (1) nach einem der Ansprüche 1 bis 4, wobei das erste Führungselement (90) und das zweite Führungselement (100) definieren:
- einen ersten Anschlagbereich (72), der dazu konfiguriert ist, mit dem Vorsprung (61) in der Ausgangsposition des hinteren Schutzschilds (30) in Eingriff zu treten, um ein Längsgleiten des hinteren Schutzschilds (30) in Richtung des proximalen Endes (22) der Nadel (20) zu verhindern;
- einen zweiten Anschlagbereich (73), der dazu konfiguriert ist, mit dem Vorsprung (61) in der Verriegelungsposition des hinteren Schutzschilds (30) in Eingriff zu treten, um ein Längsgleiten des hinteren Schutzschilds (30) sowohl in Richtung des distalen Endes (21) als auch des proximalen Endes (22) der Nadel (10) zu verhindern.

6. Sicherheitsnadelvorrichtung (1) nach Anspruch 5, wobei:
- der erste Anschlagbereich (72) und der zweite Anschlagbereich (73) proximal zur Zwischenöffnung (15) angeordnet sind;
- der hintere Schutzschild (30) in der Ausgangsposition das proximale Ende (22) der Nadel (20) abdeckt;
- der hintere Schutzschild (30) in der Zwischenposition einen ersten Abschnitt (23) der Nadel (20), der innerhalb des Hohlraums (13) angeordnet ist, und das proximale Ende (22) der Nadel (20) unbedeckt lässt;
- der hintere Schutzschild (30) in der Verriegelungsposition das proximale Ende (22) der Nadel (20) abdeckt.

7. Sicherheitsnadelvorrichtung (1) nach Anspruch 5, wobei:
- der erste Anschlagbereich (72) in Richtung des distalen Endes (21) der Nadel (20) und der zweite Anschlagbereich (73) in Richtung des proximalen Endes (22) der Nadel (20) angeordnet ist;
- der hintere Schutzschild (30) in der Ausgangsposition einen zweiten Abschnitt (24) der Nadel (20), der innerhalb des Hohlraums (13) angeordnet ist, und das proximale Ende (22) der Nadel (20) unbedeckt lässt;
- der hintere Schutzschild (30) in der Zwischenposition einen ersten Abschnitt (23) der Nadel (20), der innerhalb des Hohlraums (13) angeordnet ist, und das proximale Ende (22) der Nadel (20) unbedeckt lässt, wobei der zweite Abschnitt (24) kürzer ist als der erste Abschnitt (23);
- der hintere Schutzschild (30) in der Verriegelungsposition das proximale Ende (22) der Nadel (20) abdeckt.

8. Sicherheitsnadelvorrichtung (1) nach einem der Ansprüche 4 bis 7, wobei das erste Führungselement (90) definiert:
- einen dritten Anschlagbereich (74), der dazu konfiguriert ist, mit dem Vorsprung (61) in der Zwischenposition in Eingriff zu treten, um ein Längsgleiten des hinteren Schutzschilds (30) in Richtung des distalen Endes (22) der Nadel (20) zu verhindern;
- wobei der dritte Anschlagbereich (74) von dem ersten Anschlagbereich (72) und dem zweiten Anschlagbereich (73) entlang der Längsrichtung (X-X) in Richtung des distalen Endes (21) der Nadel (20) beabstandet ist.

9. Sicherheitsnadelvorrichtung (1) nach einem der Ansprüche 1 bis 8, wobei das erste Führungselement (90) umfasst:
- einen ersten Drehabschnitt (75), der dazu konfiguriert ist, mit dem Vorsprung (61) in Eingriff zu treten und mit dem zweiten Führungselement (100) zusammenzuwirken, um den hinteren Schutzschild (30) beim Gleiten des hinteren Schutzschilds (30) entlang der Längsachse (X-X) von der Ausgangsposition in Richtung der Zwischenposition zu drehen;
- einen zweiten Drehabschnitt (76), der dazu konfiguriert ist, mit dem Vorsprung (61) in Eingriff zu treten und mit dem zweiten Führungselement (100) zusammenzuwirken, um den hinteren Schutzschild (30) beim Gleiten des hinteren Schutzschilds (30) entlang der Längsachse (X-X) von der Zwischenposition in Richtung der Verriegelungsposition zu drehen.

10. Sicherheitsnadelvorrichtung (1) nach einem der Ansprüche 1 bis 9, wobei das erste Führungselement (90) umfasst:
- einen linearen Führungsabschnitt (77), der dazu konfiguriert ist, mit dem Vorsprung (61) in Eingriff zu treten und den hinteren Schutzschild (30) entlang der Längsachse (X-X) zu führen, wobei eine Drehung des hinteren Schutzschilds (30) von der Ausgangsposition in Richtung der Zwischenposition und von der Zwischenposition in Richtung der Verriegelungsposition verhindert wird.

11. Sicherheitsnadelvorrichtung (1) nach einem der Ansprüche 1 bis 9, wobei:
- die Nabe (10) eine Innenfläche (10a), die dem Hohlraum (13) zugewandt ist, und eine gegenüberliegende Außenfläche (10b) aufweist;
- das erste Führungselement (90) eine auf der Innenfläche (10a) ausgebildete Nut umfasst;
- die Vorsprünge (61) Stifte sind;
- das elastische Element (40) ein Federelement (41) umfasst, das sich zwischen einem distalen Abschnitt (42) und einem proximalen Abschnitt (43) entlang der Längsachse (X-X) erstreckt.

12. Sicherheitsnadelvorrichtung (1) nach einem der Ansprüche 1 bis 11, wobei der hintere Schutzschild (30) einen Hohlkörper (31) umfasst, der sich zwischen einem distalen Abschnitt (30a), der der oberen Wand (12) der Nabe (10) zugewandt ist, und einem proximalen Abschnitt (30b) erstreckt, der der unteren Öffnung (14) der Nabe (10) zugewandt ist, wobei der Hohlkörper (31) umfasst:
- eine untere Wand (32), die proximal zum distalen Abschnitt (30a) ausgebildet ist und eine Öffnung (33) aufweist, die dazu konfiguriert ist, einem Abschnitt der Nadel (20) und dem proximalen Ende (22) der Nadel (20) den Durchtritt zu ermöglichen, wenn sich der hintere Schutzschild (30) zwischen der Ausgangsposition und der Verriegelungsposition bewegt;
- eine Seitenwand (35), die sich von der unteren Wand (32) entlang der Längsachse (X-X) erstreckt und einen Durchgangskanal (34) definiert, der dazu konfiguriert ist, die Nadel (20) zumindest teilweise aufzunehmen;
- eine Haltewand (38), die sich entlang der Längsrichtung X-X von der unteren Wand (32) in Richtung des distalen Endes der Nadel (20) erstreckt und einen Abschnitt der Seitenwand (35) umgibt.

13. Sicherheitsnadelvorrichtung (1) nach Anspruch 12, wobei die Haltewand (38) eine Innenfläche (38a), die dem umgebenen Abschnitt der Seitenwand (35) zugewandt ist, und eine Außenfläche (38b) aufweist, die dem Hohlraum (13) der Nabe (10) zugewandt ist, und die Vorsprünge (61) auf der Außenfläche (38b) des hinteren Schutzschilds (30) ausgebildet sind.

14. Sicherheitsnadelvorrichtung (1) nach Anspruch 12 oder 13, wobei:
- das elastische Element (40) zwischen der oberen Wand (12) der Nabe (10) und der unteren Wand (32) des hinteren Schutzschilds (30) angeordnet ist;
- das elastische Element (40) die Nadel (20), die durch den Hohlraum (13) der Nabe (10) verläuft, zumindest teilweise umgibt.

15. Sicherheitsnadelvorrichtung (1) nach einem der Ansprüche 1 bis 14, wobei
- die Nabe (10) zwei symmetrische Führungsbahnen (71) umfasst, die in Umfangsrichtung auf der Seitenwand (11) beabstandet sind;
- der hintere Schutzschild (30) zwei symmetrische Vorsprünge (61) umfasst, die in Umfangsrichtung auf dem hinteren Schutzschild (30) beabstandet sind,
- das zweite Führungselement (100) zwei symmetrische Führungsflügel (140) umfasst, die in Umfangsrichtung auf dem ringförmigen Abschnitt (110) beabstandet sind.

## Revendications

1. Dispositif d'aiguille de sécurité (1) comprenant :
- un moyeu (10) configuré pour être connecté à un stylo injecteur, le moyeu (10) comprenant une paroi latérale (11) et une paroi supérieure (12) qui définissent entre elles une cavité (13) s'étendant entre la paroi supérieure (12) et une ouverture inférieure (14) apte à recevoir le stylo injecteur ;
- une aiguille (20) fixée au moyeu (10), l'aiguille (20) s'étendant le long d'une direction longitudinale (X-X) entre une extrémité distale (21), configurée pour être insérée dans un patient, et une extrémité proximale opposée (22) configurée pour être connectée au stylo injecteur et agencée à l'intérieur de la cavité (13) ;
- un protecteur d'extrémité arrière (30) coulissant le long de la direction longitudinale (X-X) à l'intérieur de la cavité (13) du moyeu (10) et rotatif autour de la direction longitudinale (X-X), le protecteur d'extrémité arrière (30) étant mobile de manière irréversible d'une position initiale, avant la connexion du stylo injecteur au moyeu (10), à une position de verrouillage, après le retrait du stylo injecteur du moyeu (10), en passant par une position intermédiaire ;
- un élément élastique (40) agencé à l'intérieur de la cavité (13) et agissant entre la paroi supérieure (12) et le protecteur d'extrémité arrière (30) pour solliciter le protecteur d'extrémité arrière (30) vers l'extrémité proximale (21) de l'aiguille (10) le long de la direction longitudinale (X-X) ;
- un agencement de verrouillage (50) comprenant au moins un premier élément de verrouillage (60) formé sur le protecteur d'extrémité arrière (30) et au moins un second élément de verrouillage (70), le second élément de verrouillage (70) coopérant avec le premier élément de verrouillage (60) pour guider le protecteur d'extrémité arrière (30) de la position initiale à la position de verrouillage et pour verrouiller le protecteur d'extrémité arrière (30) dans la position de verrouillage,
dans lequel
- le second élément de verrouillage (70) comprend un premier élément de guidage (90) formé sur la paroi latérale (11) du moyeu (10) et un second élément de guidage (100) fixé à la paroi latérale (11) à l'intérieur de la cavité (13) au niveau d'une ouverture intermédiaire (15) définie entre la paroi supérieure (12) et l'ouverture inférieure (14), le premier élément de guidage (90) et le second élément de guidage (100) définissant une piste de guidage (71) à l'intérieur de la cavité (13) ;
- le premier élément de verrouillage (60) comprend une saillie (61) configurée pour s'engager avec le premier élément de guidage (90) et le second élément de guidage (100) pour coulisser le long de la piste de guidage (71).

2. Dispositif d'aiguille de sécurité (1) selon la revendication 1, dans lequel le second élément de guidage (100) comprend :
- une portion annulaire (110) configurée pour s'engager avec la paroi latérale (11) au niveau de l'ouverture intermédiaire (15) et définissant un canal de passage (120) pour permettre le coulissement du protecteur d'extrémité arrière (30) le long de la direction longitudinale (X-X) ;
- une aile de guidage (140) se projetant depuis la portion annulaire (110) vers l'extrémité distale (21) à l'intérieur de la cavité (13) et configurée pour s'engager avec la saillie (61) et la guider de la position initiale à la position de verrouillage.

3. Dispositif d'aiguille de sécurité (1) selon la revendication 2, dans lequel l'aile de guidage (140) comprend :
- une première portion d'aile de rotation (141) configurée pour coopérer avec le premier élément de guidage (90) pour guider et faire tourner le protecteur d'extrémité arrière (30) lors du coulissement dudit protecteur d'extrémité arrière (30) le long de l'axe longitudinal (X-X) de la position initiale vers la position intermédiaire ;
- une seconde portion d'aile de rotation (142) configurée pour coopérer avec le premier élément de guidage (90) pour guider et faire tourner le protecteur d'extrémité arrière (30) lors du coulissement dudit protecteur d'extrémité arrière (30) le long de l'axe longitudinal (X-X) de la position intermédiaire vers la position de verrouillage.

4. Dispositif d'aiguille de sécurité (1) selon la revendication 2 ou 3, dans lequel la portion annulaire (110) possède des moyens de retenue (150) configurés pour s'engager avec la paroi latérale (12) au niveau de l'ouverture intermédiaire (15).

5. Dispositif d'aiguille de sécurité (1) selon l'une quelconque des revendications 1 à 4, dans lequel le premier élément de guidage (90) et le second élément de guidage (100) définissent :
- une première zone d'arrêt (72) configurée pour s'engager avec la saillie (61) dans la position initiale du protecteur d'extrémité arrière (30) afin d'empêcher un coulissement longitudinal du protecteur d'extrémité arrière (30) vers l'extrémité proximale (22) de l'aiguille (20) ;
- une seconde zone d'arrêt (73) configurée pour s'engager avec la saillie (61) dans la position de verrouillage du protecteur d'extrémité arrière (30) afin d'empêcher un coulissement longitudinal du protecteur d'extrémité arrière (30) à la fois vers l'extrémité distale (21) et vers l'extrémité proximale (22) de l'aiguille (10).

6. Dispositif d'aiguille de sécurité (1) selon la revendication 5, dans lequel :
- la première zone d'arrêt (72) et la seconde zone d'arrêt (73) sont agencées à proximité de l'ouverture intermédiaire (15) ;
- le protecteur d'extrémité arrière (30) dans la position initiale couvre l'extrémité proximale (22) de l'aiguille (20) ;
- le protecteur d'extrémité arrière (30) dans la position intermédiaire laisse découverte une première portion (23) de l'aiguille (20) agencée à l'intérieur de la cavité (13) et l'extrémité proximale (22) de l'aiguille (20) ;
- le protecteur d'extrémité arrière (30) dans la position de verrouillage couvre l'extrémité proximale (22) de l'aiguille (20).

7. Dispositif d'aiguille de sécurité (1) selon la revendication 5, dans lequel :
- la première zone d'arrêt (72) est agencée vers l'extrémité distale (21) de l'aiguille (20) et la seconde zone d'arrêt (73) est agencée vers l'extrémité proximale (22) de l'aiguille (20) ;
- le protecteur d'extrémité arrière (30) dans la position initiale laisse découverte une seconde portion (24) de l'aiguille (20) agencée à l'intérieur de la cavité (13) et l'extrémité proximale (22) de l'aiguille (20) ;
- le protecteur d'extrémité arrière (30) dans la position intermédiaire laisse découverte une première portion (23) de l'aiguille (20) agencée à l'intérieur de la cavité (13) et l'extrémité proximale (22) de l'aiguille (20), la seconde portion (24) étant plus courte que la première portion (23) ;
- le protecteur d'extrémité arrière (30) dans la position de verrouillage couvre l'extrémité proximale (22) de l'aiguille (20).

8. Dispositif d'aiguille de sécurité (1) selon l'une quelconque des revendications 4 à 7, dans lequel le premier élément de guidage (90) définit :
- une troisième zone d'arrêt (74) configurée pour s'engager avec la saillie (61) dans la position intermédiaire pour empêcher un coulissement longitudinal du protecteur d'extrémité arrière (30) vers l'extrémité distale (22) de l'aiguille (20) ;
- la troisième zone d'arrêt (74) étant espacée de la première zone d'arrêt (72) et de la seconde zone d'arrêt (73) le long de la direction longitudinale (X-X) vers l'extrémité distale (21) de l'aiguille (20).

9. Dispositif d'aiguille de sécurité (1) selon l'une quelconque des revendications 1 à 8, dans lequel le premier élément de guidage (90) comprend :
- une première portion de rotation (75) configurée pour s'engager avec la saillie (61) et coopérant avec le second élément de guidage (100) pour faire tourner le protecteur d'extrémité arrière (30) lors du coulissement dudit protecteur d'extrémité arrière (30) le long de l'axe longitudinal (X-X) de la position initiale vers la position intermédiaire ;
- une seconde portion de rotation (76) configurée pour s'engager avec la saillie (61) et coopérant avec le second élément de guidage (100) pour faire tourner le protecteur d'extrémité arrière (30) lors du coulissement dudit protecteur d'extrémité arrière (30) le long de l'axe longitudinal (X-X) de la position intermédiaire vers la position de verrouillage.

10. Dispositif d'aiguille de sécurité (1) selon l'une quelconque des revendications 1 à 9, dans lequel le premier élément de guidage (90) comprend :
- une portion de guidage linéaire (77) configurée pour s'engager avec la saillie (61) et guider le protecteur d'extrémité arrière (30) le long de l'axe longitudinal (X-X) en empêchant la rotation du protecteur d'extrémité arrière (30) de la position initiale vers la position intermédiaire et de la position intermédiaire vers la position de verrouillage.

11. Dispositif d'aiguille de sécurité (1) selon l'une quelconque des revendications 1 à 9, dans lequel :
- le moyeu (10) a une surface intérieure (10a) faisant face à la cavité (13) et une surface extérieure opposée (10b) ;
- le premier élément de guidage (90) comprend une rainure formée sur la surface intérieure (10a) ;
- les saillies (61) sont des broches ;
- l'élément élastique (40) comprend un élément de ressort (41) s'étendant entre une portion distale (42) et une portion proximale (43) le long de l'axe longitudinal (X-X).

12. Dispositif d'aiguille de sécurité (1) selon l'une quelconque des revendications 1 à 11, dans lequel le protecteur d'extrémité arrière (30) comprend un corps creux (31) s'étendant entre une portion distale (30a) faisant face à la paroi supérieure (12) du moyeu (10) et une portion proximale (30b) faisant face à l'ouverture inférieure (14) du moyeu (10), le corps creux (31) comprenant :
- une paroi inférieure (32) formée à proximité de la portion distale (30a) et ayant une ouverture (33) configurée pour permettre à une portion de l'aiguille (20) et à l'extrémité proximale (22) de l'aiguille (20) de passer lors du déplacement du protecteur d'extrémité arrière (30) entre la position initiale et la position de verrouillage ;
- une paroi latérale (35) s'étendant depuis la paroi inférieure (32) le long de l'axe longitudinal (X-X) et définissant un canal de passage (34) configuré pour loger au moins partiellement l'aiguille (20) ;
- une paroi de retenue (38) s'étendant le long de la direction longitudinale X-X depuis la paroi inférieure (32) vers l'extrémité distale de l'aiguille (20) et entourant une portion de la paroi latérale (35).

13. Dispositif d'aiguille de sécurité (1) selon la revendication 12, dans lequel la paroi de retenue (38) a une surface intérieure (38a) faisant face à la portion entourée de la paroi latérale (35) et une surface extérieure (38b) faisant face à la cavité (13) du moyeu (10) et les saillies (61) sont formées sur la surface extérieure (38b) du protecteur d'extrémité arrière (30).

14. Dispositif d'aiguille de sécurité (1) selon la revendication 12 ou 13, dans lequel :
- l'élément élastique (40) est interposé entre la paroi supérieure (12) du moyeu (10) et la paroi inférieure (32) du protecteur d'extrémité arrière (30) ;
- l'élément élastique (40) entoure au moins partiellement l'aiguille (20) passant à l'intérieur de la cavité (13) du moyeu (10).

15. Dispositif d'aiguille de sécurité (1) selon l'une quelconque des revendications 1 à 14, dans lequel
- le moyeu (10) comprend deux pistes de guidage symétriques (71) espacées circonférentiellement sur la paroi latérale (11) ;
- le protecteur d'extrémité arrière (30) comprend deux saillies symétriques (61) espacées circonférentiellement sur le protecteur d'extrémité arrière (30),
- le second élément de guidage (100) comprend deux ailes de guidage symétriques (140) espacées circonférentiellement sur la portion annulaire (110).
